(19) Europäisches Patentamt
European Patent Office
Office européen des brevets

(11) Veröffentlichungsnummer: **0 337 560 A2**

(12) # EUROPÄISCHE PATENTANMELDUNG

(21) Anmeldenummer: **89200882.2**

(51) Int. Cl.4: **C07D 213/71 , A01N 43/40**

(22) Anmeldetag: **30.03.89**

(30) Priorität: **13.04.88 DE 3812177**

(43) Veröffentlichungstag der Anmeldung:
**18.10.89 Patentblatt 89/42**

(84) Benannte Vertragsstaaten:
**BE CH DE FR GB IT LI NL SE**

(71) Anmelder: **BAYER AG**

**D-5090 Leverkusen 1 Bayerwerk(DE)**

(72) Erfinder: **Klausener, Alexander, Dr.**
**Bendenstrasse 16**
**D-5190 Stolberg(DE)**
Erfinder: **Paulus, Wilfried, Dr.**
**Deswatinesstrasse 90**
**D-4150 Krefeld 1(DE)**
Erfinder: **Schmitt, Hans-Georg, Dr.**
**Am Oberend 13**
**D-4150 Krefeld 1(DE)**
Erfinder: **Brandes, Wilhelm, Dr.**
**Eichendorffstrasse 3**
**D-5653 Leichlingen 1(DE)**

(54) **2-Phenylsulfinyl-nitro-pyridine, Verfahren zu ihrer Herstellung und ihre Verwendung.**

(57) Die Erfindung betrifft 2-Phenylsulfinyl-nitro-pyridine der Formel

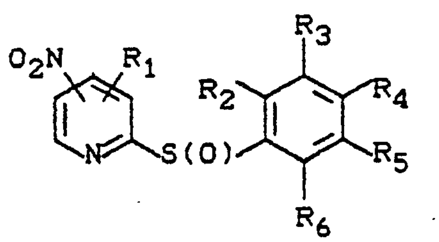

(I),

in der
$R_1$ Wasserstoff, Alkyl oder Halogen bedeutet und
$R_2$,$R_3$,$R_4$,$R_5$ und $R_6$ unabhängig voneinander für Wasserstoff, Halogen, Alkyl, Haloalkyl, Alkoxy, Haloalkoxy, Cyano, Nitro, Carboxy, Alkoxycarbonyl, Carbonamido, N-Alkyl- oder N,N-Dialkylcarbonamido, Acyl oder primäres, sekundäres oder tertiäres Amino stehen.
Verfahren zu ihrer Herstellung und ihre Verwendung als Mikrobizide für den Materialschutz und Fungizide im Pflanzenschutz.

EP 0 337 560 A2

## 2-Phenylsulfinyl-nitro-pyridine, Verfahren zu ihrer Herstellung und ihre Verwendung

Die Erfindung betrifft neue Derivate des Nitropyridins, Verfahren zu ihrer Herstellung und ihre Verwendung als Mikrobizide für den Materialschutz.

Aus der US-PS 3 296 272 ist bereits bekannt. Alkylsulfinyl- und Alkylsulfonylpolyhalo-pyridine als Mikrobizide zum Schutz technischer Materialien zu verwenden. Die antimikrobielle Wirksamkeit dieser Alkylsulfinyl-und Alkylsulfonyl-pyridine ist jedoch auf bestimmten Indikationsgebieten nicht befriedigend; insbesondere macht sich ihre Hydrolyseempfindlichkeit bei ihrer Anwendung in wäßrigen Systemen nachteilig bemerkbar, z.B. bei ihrer Anwendung als Anstrichfungizide in Dispersionsfarben, insbesondere wenn diese alkalisch reagieren (siehe z.B. die Angaben in der DE-OS 2 048 354, S. 14).

Überraschenderweise wurde gefunden, daß Nitropyridine, die in 2-Stellung durch eine Phenylsulfinyl-Gruppe substituiert sind, eine wesentlich verbesserte mikrobizide Wirkung, ein wesentlich breiteres Wirkungsspektrum und eine wesentlich erhöhte Hydrolysestabilität aufweisen und sich deshalb sehr viel besser für eine Verwendung als Mikrobizide im Materialschutz eignen als die durch Alkylsulfinyl- bzw. Alkylsulfonyl-Gruppen substituierten Halo-pyridine. Diese in 2-Stellung durch eine Phenylsulfinylgruppe substituierten Nitropyridine sind neu.

Die Erfindung betrifft daher 2-Phenylsulfinyl-nitro-pyridine der Formel

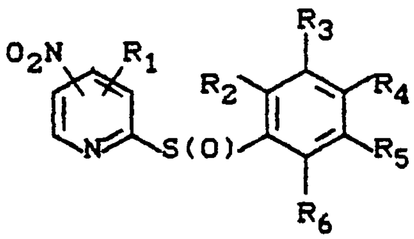

(I),

in der

$R_1$ Wasserstoff, Alkyl oder Halogen bedeutet und

$R_2$,$R_3$,$R_4$,$R_5$ und $R_6$ unabhängig voneinander für Wasserstoff, Halogen, Alkyl, Haloalkyl, Alkoxy; Haloalkoxy, Cyano, Nitro, Carboxy, Alkoxycarbonyl, Carbonamido, N-Alkyl- oder N,N-Dialkylcarbonamido, Acyl oder primäres, sekundäres oder tertiäres Amino stehen.

Bevorzugt sind die Verbindungen der Formel (I), in der

$R_1$ für Wasserstoff und

$R_2$,$R_3$,$R_4$,$R_5$ und $R_6$ unabhängig voneinander für Wasserstoff, Halogen, Alkyl, Haloalkyl, Alkoxy, Haloalkoxy, Cyano oder Nitro stehen.

Besonders bevorzugt sind die Verbindungen der Formel (I), wenn in dieser die $NO_2$-Gruppe in 3-, 4- oder 5-Stellung, ganz besonders bevorzugt in 5-Stellung, steht,

$R_1$ Wasserstoff ist und

$R_2$, $R_5$, $R_6$ unabhängig voneinander Halogen, Alkyl, Halogenalkyl oder vorzugsweise Wasserstoff und

$R_3$ und $R_4$ unabhängig voneinander Wasserstoff, Halogen, Alkyl oder Halogenalkyl, bedeuten.

Als Vertreter der erfindungsgemäßen 2-Arylsulfinylnitropyridine der Formel (I) seien beispielsweise die Verbindungen genannt, in denen $R_1$, $R_2$, $R_3$, $R_4$, $R_5$ und $R_6$ die in Tabelle 1 angegebene Bedeutung und die Nitrogruppe, die ebenfalls in Tabelle 1 angegebene Stellung haben.

Tabelle 1

| $R_1$ | $NO_2$ | $R_2$ | $R_3$ | $R_4$ | $R_5$ | $R_6$ |
|---|---|---|---|---|---|---|
| 6-Cl | 3 | H | H | $N(CH_3)_2$ | H | H |
| H | 3 | H | $OC_3H_7i$ | H | H | H |
| H | 3 | H | CN | H | H | H |
| 5-$CH_3$ | 4 | H | H | $OCF_3$ | H | H |
| 6-$CH_3$ | 4 | F | H | Br | H | H |
| 6-$C_3H_7i$ | 4 | H | Cl | H | H | H |
| 6-Cl | 5 | H | $COOCH_3$ | H | H | H |
| H | 5 | Cl | H | $CF_3$ | H | H |
| H | 5 | H | $CH_3$ | H | H | H |
| H | 5 | H | H | $CON(CH_3)_2$ | H | H |
| H | 5 | $OCH_3$ | H | H | H | H |
| H | 5 | Cl | Cl | Cl | Cl | Cl |
| 4-t-$C_4H_9$ | 5 | H | $CF_3$ | H | H | Cl |
| H | 5 | H | H | t-$C_4H_9$ | H | H |
| H | 5 | H | H | $COOC_4H_9n$ | H | H |
| H | 5 | H | H | $NO_2$ | H | H |
| H | 6 | H | Cl | H | H | H |
| H | 6 | H | $CF_3$ | H | H | H |
| H | 6 | H | H | COOH | H | H |
| H | 5 | H | H | $CF_3$ | H | H |
| 3-Cl | 5 | H | H | Cl | H | H |

Die erfindungsgemäßen 2-Phenylsulfinyl-nitro-pyridine der Formel (I) werden durch Oxidation der entsprechenden 2-Phenylmercapto-nitro-pyridine erhalten. Die Erfindung betrifft daher auch ein Verfahren zur Herstellung der 2-Phenylsulfinyl-nitro-pyridine der Formel (I), das dadurch gekennzeichnet ist, daß man 2-Phenylmercapto-nitro-pyridine der Formel

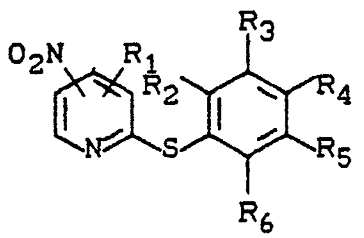

(II),

in der

$R_1$, $R_2$, $R_3$, $R_4$, $R_5$ und $R_6$ die unter Formel (I) angegebene Bedeutung haben,

nach für die selektive Oxidation von Diarylsulfiden bzw. Hetaryl-aryl-sulfiden an sich bekannten Verfahren oxidiert.

Als Oxidationsmittel werden in dem erfindungsgemäßen Verfahren z.B. Salpetersäure, $H_2O_2$ und, vorzugsweise, die Acylderivate des $H_2O_2$, die organischen Persäuren, verwendet. Vorzugsweise wird als Oxidationsmittel m-Chlor-peroxybenzoesäure eingesetzt.

Die Oxidationsmittel werden in einer solchen Menge verwendet, daß auf 1 Äquivalent Mercapto-Verbindung der Formel (II) 0,8 bis 1,2 Äquivalente Persäure, vorzugsweise 1 Äquivalent Persäure entfällt.

Die Oxidation wird bevorzugt in einem organischen, unter den Reaktionsbedingungen inerten Lösungsmittel vorgenommen. Als inerte Lösungsmittel seien vor allem halogenierte Kohlenwasserstoffe wie Dichlormethan, Trichlormethan, Tetrachlormethan, Trichlorethan oder Tetrachlorethan genannt; aber auch aliphatische oder aromatische Kohlenwasserstoffe wie Cyclohexan, Benzol, Toluol oder Xylol sowie Ether, wie Diethylether, Tetrahydrofuran oder Dioxan, kommen als Lösungsmittel in Betracht.

Die Reaktion wird üblicherweise unter Normaldruck ausgeführt, sie kann aber auch unter vermindertem oder erhöhtem Druck durchgeführt werden.

Die Oxidation wird vorzugsweise bei Temperaturen von -80 bis +100°C, bevorzugt bei -20 bis +20°C

ausgeführt.

Die als Ausgangsverbindungen für die Oxidation erforderlichen 2-Phenylmercapto-nitro-pyridine der Formel (II) sind bekannt oder lassen sich nach bekannten Herstellungsvorschriften darstellen (siehe z.B. E. Klingsberg, "Pyridine and Its Derivatives" Part IV Interscience Publishers, J. Wiley & Sons, New York-London-Sydney (1964), R.A. Abramovich (Ed.), "Pyridine and Its Derivatives" Supplement Part IV, J. Wiley & Sons, New York-London-Toronto (1975)).

Die Erfindung betrifft ferner die Verwendung der 2-Phenylsulfinyl-5-nitro-pyridine der Formel (I) als Mikrobizide zum Schutz technischer Materialien.

Bei den erfindungsgemäß zu schützenden technischen Materialien handelt es sich um nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, deren Funktion durch Vermehrung von Mikroorganismen beeinträchtigt werden kann. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz dauerhaft verfärbende und holzzerstörende Pilze, sowie gegen Schleimorganismen und Algen.

Es seien beispielsweise Mikroorganismen der folgenden Gattungen genannt:

Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Je nach Anwendungsgebiet können die erfindungsgemäß zu verwendenden Wirkstoffe in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Die zum Schutz technischer Materialien verwendeten mikrobiziden Mittel enthalten die Wirkstoffe im allgemeinen in einer Menge von 1 bis 95 Gew.-%, bevorzugt von 10 bis 75 Gew.-%.

Die Anwendungskonzentrationen der erfindungsgemäß zu verwendenden Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäß zu verwendenden Wirkstoffe können auch in Mischung mit anderen bekannten Wirkstoffen angewendet werden. Beispielsweise seien die folgenden Wirkstoffe genannt: Benzylalkoholmono(poly)hemiformal und andere Formaldehyd abspaltende Verbindungen, Benzimidazolylmethylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Thiocyanatomethylthiobenzthiazol. Methylenbisthiocyanat, Phenolderivate, wie 2-Phenylphenol, (2,2′-Dihydroxy-5,5′-dichlor)-diphenylmethan und 3-Methyl-4-chlor-phenol, Organo-Zinnverbindungen, N-Trihalogenmethylthio-verbindungen, wie Folpet, Fluorfolpet und Dichlofluanid, Azolfungizide wie Triadimefon, Triadimenol und Bitertanol.

Die erfindungsgemäßen 2-Phenylsulfinyl-nitro-pyridine der Formel I zeichnen sich aber nicht nur durch ein breites Wirkungsspektrum gegenüber technische Materialien befallende Mikroorganismen aus, sondern weisen fernerhin auch eine gute fungizide Wirkung gegen Pyricularia oryzae an Reis und eine gute in vitro-Wirkung gegen phytopathogene Pilze auf. Sie können deshalb auch mit gutem Erfolg im Pflanzenschutz gegen diese Schadmikroben eingesetzt werden.

Beispiel 1

Die Lösung von 80 g (0,3 Mol) 5-Nitro-2-(4-chlorphenyl)-mercapto-pyridin in 1000 ml Dioxan wird bei 0°C unter Rühren und Kühlen portionsweise mit insgesamt 60,9 g (0,3 Mol) m-Chlorperoxybenzoesäure (95 %ig) versetzt. Nach beendeter Zugabe läßt man das Reaktionsgemisch sich auf Raumtemperatur erwärmen, rührt es 12 Stunden bei dieser Temperatur und versetzt es anschließend mit 55 g pulverförmigem wasserfreiem Natriumcarbonat. Anschließend wird das Reaktionsgemisch noch etwa 4 Stunden gerührt. Nach dem Abfiltrieren des Niederschlags und Auswaschen mit Dichlormethan werden Filtrat und Waschlösungen vereinigt, eingeengt und der Rückstand aus Ethanol/Diisopropylether umkristallisiert.
Ausbeute: 56,1 g (= 66,1 % der Theorie)
Fp.: 157 bis 159°C

Das als Ausgangsverbindung verwendete 5-Nitro-2-(4-chlorphenyl)-mercapto-pyridin war wie folgt erhalten worden:

Die Lösung von 79,3 g (0,5 Mol) 2-Chlor-5-nitro-pyridin in 500 ml Dioxan wurde mit 150 g pulverförmigem wasserfreiem Kaliumcarbonat und 76,7 g (0,52 Mol) 4-Chlorthiophenol versetzt. Die Reaktionsmischung wurde unter Stickstoff und Rühren auf 100°C erhitzt und etwa 6 Stunden auf dieser Temperatur gehalten. Nach dem Abkühlen wurde mit das Reaktionsgemisch mit Dichlormethan verdünnt und mit Wasser gewaschen. Die organische Phase wurde mit wasserfreiem Natriumsulfat getrocknet und anschließend im Vakuum eingeengt. Der Rückstand wurde aus Toluol/n-Hexan umkristallisiert.
Ausbeute: 111,2 g (83,3 % der Theorie)
Fp.: 133 bis 135°C

In gleicher Weise wurden die 2-Phenylsulfinyl-nitropyridine der Formel (I) hergestellt, in der $R_1$, $R_2$, $R_3$, $R_4$, $R_5$ und $R_6$ die in Tabelle 2 angegebene Bedeutung und die Nitrogruppe die ebenfalls in Tabelle 2 angegebene Stellung haben.

Tabelle 2

| Beispiel | $R_1$ | $NO_2$ | $R_2$ | $R_3$ | $R_4$ | $R_5$ | $R_6$ | Fp. (°C) |
|---|---|---|---|---|---|---|---|---|
| 2 | H | 5-$NO_2$ | H | $CF_3$ | H | H | H | 95-100 |
| 3 | H | 5-$NO_2$ | H | H | H | H | H | 152-5 |
| 4 | H | 5-$NO_2$ | H | H | Br | H | H | 164 |
| 5 | H | 5-$NO_2$ | H | H | $CH_3$ | H | H | 112-3 |
| 6 | H | 5-$NO_2$ | H | H | F | H | H | 144-9 |
| 7 | H | 5-$NO_2$ | H | $CF_3$ | Cl | H | H | 138-40 |
| 8 | H | 5-$NO_2$ | Cl | $CF_3$ | H | H | H | 131-3 |
| 9 | H | 5-$NO_2$ | Cl | $CF_3$ | Cl | H | H | 114-7 |
| 10 | H | 5-$NO_2$ | Cl | H | Cl | $CF_3$ | H | 144-5 |
| 11 | H | 5-$NO_2$ | Cl | H | Cl | Cl | H | 164-5 |
| 12 | H | 5-$NO_2$ | H | Cl | H | H | H | 142-4 |
| 13 | H | 5-$NO_2$ | H | Cl | H | Cl | H | 154-7 |
| 14 | H | 3-$NO_2$ | H | $CF_3$ | H | H | H | 120 |
| 15 | H | 3-$NO_2$ | H | H | H | H | H | 123-6 |
| 16 | H | 3-$NO_2$ | H | H | Br | H | H | 137-41 |
| 17 | H | 3-$NO_2$ | H | H | $CH_3$ | H | H | 140-1 |
| 18 | H | 3-$NO_2$ | H | Cl | H | H | H | 102-5 |
| 19 | H | 4-$NO_2$ | H | H | Cl | H | H | Sirup |
| 20 | H | 4-$NO_2$ | H | Cl | H | H | H | 77-78 |
| 21 | H | 4-$NO_2$ | H | $CF_3$ | H | H | H | Sirup |

## Anwendungsbeispiele

### Beispiel A

Zum Nachweis der Wirksamkeit gegen Pilze werden die minimalen Hemm-Konzentrationen (MHK) von erfindungsgemäßen Wirkstoffen bestimmt:

Ein Agar, der aus Bierwürze und Pepton hergestellt wird, wird mit erfindungsgemäßen Wirkstoffen in Konzentrationen von 0,1 mg/1 bis 5000 mg/l versetzt. Nach Erstarren des Agars erfolgt Kontamination mit Reinkulturen der in der Tabelle aufgeführten Testorganismen. Nach 2-wöchiger Lagerung bei 28°C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt; sie ist in der nachstehenden Tabelle 3 angegeben.

**Tabelle 3** MHK's in mg/l bei der Einwirkung erfindungsgemäßer Substanzen auf Pilze

| Testorganismen | 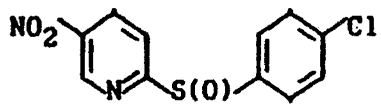 | 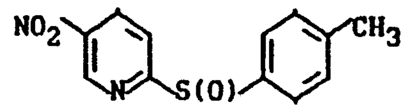 | 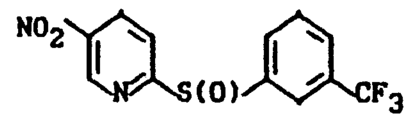 |
|---|---|---|---|
| Alternaria tenuis | 20 | 35 | 75 |
| Aspergillus niger | 50 | 150 | 100 |
| Aureobasidium pullulans | 50 | 50 | 75 |
| Chaetomium globosum | 20 | 30 | 50 |
| Cladosporium cladosporioides | 5 | 35 | 75 |
| Lentinus tigrinus | 2 | 20 | 10 |
| Penicillium glaucum | 50 | 100 | 75 |
| Sclerophoma pityophila | 10 | 5 | 7,5 |

Beispiel B (Wirkung gegen Schleimorganismen)

Erfindungsgemäße Substanzen werden in Konzentrationen von jeweils 0,1 bis 100 mg/l in Allens Nährlösung (Arch. Mikrobiol. 17, 34 bis 53 (1952)), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3-wöchiger Kultur bei Raumtemperatur noch völlig klar, d.h. die in wirkstofffreien Nährlösungen nach 3 bis 4 Tagen bemerkbare starke Vermehrung der Mikroben und Schleimbildung unterbleibt.

**Tabelle 4**

**MHK-Werte in mg/l bei der Einwirkung der unten angegebenen Substanz auf Schleimoganismen**

| Wirkstoff | MHK in mg/l |
| --- | --- |
| 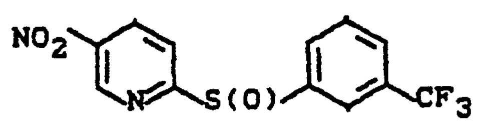 | 15 |

Beispiel C

Prüfung der Schimmelfestigkeit von Anstrichen

Die Prüfung wird in Anlehnung an den Report 219 der Defense Standards Laboratories Maribyrnong/Australien wie folgt durchgeführt:

Das zu prüfende Anstrichmittel wird beidseitig auf eine geeignete Unterlage gestrichen.

Um praxisnahe Ergebnisse zu erhalten wird ein Teil der Prüflinge vor dem Test auf Schimmelfestigkeit mit fließendem Wasser (24 Std.; 20° C) ausgelaugt; ein anderer Teil wird mit einem warmen Frischluftstrom behandelt (7 Tage; 40° C).

Die so vorbereiteten Prüflinge werden auf einen Agar-Nährboden gelegt. prüfling und Nährboden werden mit Pilzsporen kontaminiert. Nach 1- bis 3-wöchiger Lagerung bei 20 ± 1° C und 80 bis 90 % rel. Luftfeuchte wird abgemustert. Der Anstrich ist dauerhaft schimmelfest, wenn der Prüfling pilzfrei bleibt oder höchstens einen geringen Randbefall erkennen läßt.

Zur Kontamination werden Pilzsporen folgender neun Schimmelpilze verwendet, die als Anstrichzerstörer bekannt sind oder häufig auf Anstrichen angetroffen werden:

1. Alternaria tenuis
2. Aspergillus flavus
3. Aspergillus niger
4. Aspergillus ustus
5. Cladosporium herbarum
6. Paecilomyces variotii
7. Penicillium citrinum
8. Aureobasidium pullulans

9. Stachybotrys atra Corda

In Proben einer handelsüblichen Dispersionsfarbe auf Basis von Polyvinylacetat wurden, bezogen auf Gesamtfeststoffgehalt, 0 bis 1,0 Gew.-% 2-(3-Trifluormethylphenyl)-sulfonyl-5-nitro-pyridin (I) homogen eingearbeitet.

Zum Vergleich wurden Proben bereitet, die 0 bis 2 Gew.-% 4-Methylsulfonyl-2,3,5,6-tetrachlorpyridin (II) (unter die US-PS fallendes Handelsprodukt) enthalten.

Aus den Anstrichmittelproben werden Anstriche hergestellt und nach der oben beschriebenen Methode auf Schimmelfestigkeit geprüft.

Ergebnis:

Anstrichmittelproben, die 0,6-1 Gew.-% (I) enthielten, lieferten Anstriche, die auch unter den oben beschriebenen Prüfbedingungen hervorragend schimmelfest waren.

Hingegen wurden selbst mit 2 Gew.-% (II) enthaltenden Anstrichmittelproben keine schimmelfesten Anstriche erhalten.

Beispiel D

Plasmopara-Test (Reben) / protektiv

| | | |
|---|---|---|
| Lösungsmittel: | 4,7 | Gewichtsteile Aceton |
| Emulgator: | 0,3 | Gewichtsteile Alkyl-aryl-polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 22 °C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Eine deutliche Überlegenheit in der Wirksamkeit gegenüber dem Stand der Technik zeigt in diesem Test z.B. die Verbindung gemäß Herstellungsbeispiel 16.

## Tabelle 5

**Plasmopara-Test (Reben) / protektiv**

| Wirkstoff | Wirkungsgrad in % der unbehandelten Kontrolle bei einer Wirkstoffkonzentration von 5 ppm |
|---|---|
| 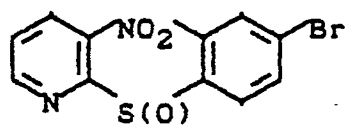 | 92 |

Beispiel E

Venturia-Test (Apfel) / protektiv

| | | |
|---|---|---|
| Lösungsmittel: | 4,7 | Gewichtsteile Aceton |
| Emulgator: | 0,3 | Gewichtsteile Alkyl-aryl-polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20° C und 100 % relativer Luftfeuchtigkeit in einer Inkubationskabine.

Die Pflanzen werden dann im Gewächshaus bei 20° C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß der Herstellungsbeispiele 2 und 11.

## Tabelle 6

**Venturia - Test (Apfel) / protektiv**

| Wirkstoff | Wirkungsgrad in % der unbehandelten Kontrolle bei einer Wirkstoffkonzentration von 5 ppm |
|---|---|
| 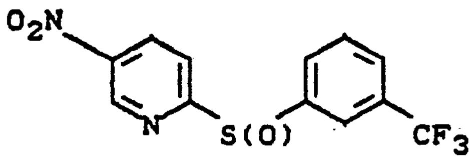 | 87 |
| 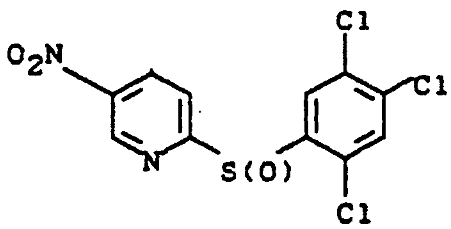 | 83 |
| zum Vergleich Fungizid gemäß Stand der Technik: | |
| 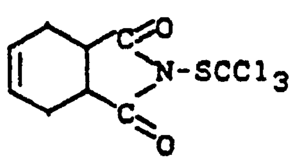 (Captan) | 54 |

## Ansprüche

1. 2-Phenylsulfinyl-nitro-pyridine der Formel

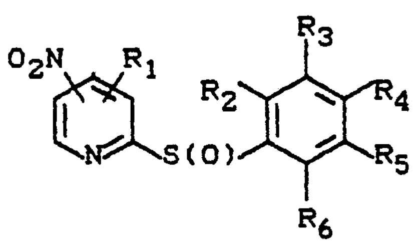

(I),

in der

$R_1$ Wasserstoff, Alkyl oder Halogen bedeutet und

$R_2$,$R_3$,$R_4$,$R_5$ und $R_6$ unabhängig voneinander für Wasserstoff, Halogen, Alkyl, Haloalkyl, Alkoxy, Haloalkoxy, Cyano, Nitro, Carboxy, Alkoxycarbonyl, Carbonamido, N-Alkyl- oder N,N-Dialkylcarbonamido, Acyl oder primäres, sekundäres oder tertiäres Amino stehen.

2. 2-Phenylsulfinyl-nitro-pyridine gemäß Anspruch 1, dadurch gekennzeichnet, daß in Formel (I) $R_1$ für Wasserstoff und $R_2$,$R_3$,$R_4$,$R_5$ und $R_6$ unabhängig voneinander für Wasserstoff, Halogen, Alkyl, Haloalkyl, Alkoxy, Haloalkoxy, Cyano oder Nitro stehen.

3. 2-Phenylsulfinyl-nitro-pyridine gemäß Anspruch 1, dadurch gekennzeichnet, daß in Formel (I) die Nitrogruppe in 3-, 4- oder 5-Stellung steht, und $R_1$ Wasserstoff und $R_2$, $R_3$, $R_4$, $R_5$ und $R_6$ unabhängig voneinander Wasserstoff, Halogen, Alkyl oder Halogenalkyl bedeuten.

4. 2-Phenylsulfinyl-nitro-pyridine gemäß Anspruch 1, dadurch gekennzeichnet, daß in Formel (I) die Nitrogruppe in 5-Stellung steht und $R_1$, $R_2$, $R_5$, $R_6$ Wasserstoff und $R_3$ und $R_4$ unabhängig voneinander Wasserstoff, Halogen, Alkyl oder Halogenalkyl bedeuten.

5. Verfahren zur Herstellung von 2-Phenylsulfinyl-nitro-pyridinen der Formel

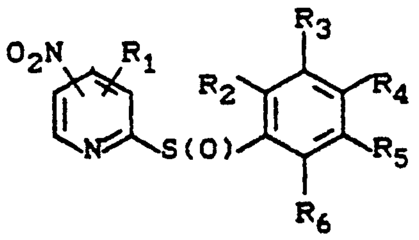

(I),

in der $R_1$ Wasserstoff, Alkyl oder Halogen bedeutet und $R_2$,$R_3$,$R_4$,$R_5$ und $R_6$ unabhängig voneinander für Wasserstoff, Halogen, Alkyl, Haloalkyl, Alkoxy, Haloalkoxy, Cyano, Nitro, Carboxy, Alkoxycarbonyl, Carbonamido, N-Alkyl- oder N,N-Dialkylcarbonamido, Acyl oder primäres, sekundäres oder tertiäres Amino stehen, dadurch gekennzeichnet, daß man 2-Phenylmercapto-nitro-pyridine der Formel

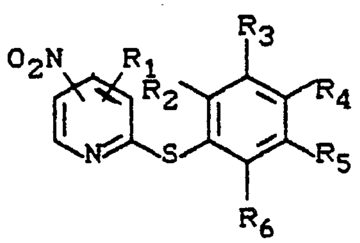

(II),

in der $R_1$,$R_2$,$R_3$,$R_4$,$R_5$ und $R_6$ die unter Formel (I) angegebene Bedeutung haben, nach für die selektive Oxidation von Diarylsulfiden bzw. Hetaryl-aryl-sulfiden an sich bekannten Verfahren oxidiert.

6. Verwendung der 2-Phenylsulfinyl-nitro-pyridine gemäß einem der Ansprüche 1 bis 5 als Mikrobizide zum Schutz technischer Materialien und als Fungizide im Pflanzenschutz.

7. Verwendung gemäß Anspruch 6 als Fungizide zum Schutz von Holz vor Holz dauerhaft verfärbenden oder holzzerstörenden Pilzen und in Anstrichmitteln.

8. Mikrobizide Mittel für den Materialschutz enthaltend als Wirkstoff 2-Phenylsulfinyl-nitro-pyridine gemäß einem der Ansprüche 1 bis 5, übliche Formulierungshilfsmittel und gegebenenfalls bekannterweise im Materialschutz verwendete Mikrobizide.